(19) Europäisches Patentamt / European Patent Office / Office européen des brevets

(11) **EP 3 593 785 B1**

(12) **EUROPEAN PATENT SPECIFICATION**

(45) Date of publication and mention
of the grant of the patent:
**26.07.2023 Bulletin 2023/30**

(21) Application number: **18763635.2**

(22) Date of filing: **05.03.2018**

(51) International Patent Classification (IPC):
**A61K 6/851** (2020.01)   **A61K 6/54** (2020.01)
**A61K 6/849** (2020.01)   **A61K 6/836** (2020.01)

(52) Cooperative Patent Classification (CPC):
**A61K 6/836; A61K 6/54; A61K 6/849; A61K 6/851**

(86) International application number:
**PCT/KR2018/002598**

(87) International publication number:
**WO 2018/164436 (13.09.2018 Gazette 2018/37)**

(54) **DENTAL COMPOSITION**

**DENTALE ZUSAMMENSETZUNG**

**COMPOSITION DENTAIRE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2017 KR 20170028689**

(43) Date of publication of application:
**15.01.2020 Bulletin 2020/03**

(73) Proprietor: **Vericom Co. Ltd.**
**Chuncheon-si, Gangwon-do 24427 (KR)**

(72) Inventors:
• **OH, Myung-Hwan**
**Seoul 05701 (KR)**
• **KANG, Jong-Ho**
**Anyang-si**
**Gyeonggi-do 14092 (KR)**
• **KIM, Yun-Ki**
**Anyang-si**
**Gyeonggi-do 14040 (KR)**

(74) Representative: **Stolmár & Partner**
**Patentanwälte PartG mbB**
**Blumenstraße 17**
**80331 München (DE)**

(56) References cited:
**JP-A- 2008 023 003      KR-A- 20060 134 223**
**KR-A- 20100 037 979      KR-A- 20150 100 446**
**KR-A- 20150 115 183      KR-A- 20150 144 028**

• **RATAN K. MISHRA ET AL: "A force field for tricalcium aluminate to characterize surface properties, initial hydration, and organically modified interfaces in atomic resolution", DALTON TRANSACTIONS, vol. 43, no. 27, 1 January 2014 (2014-01-01), pages 10602-10616, XP055742810, ISSN: 1477-9226, DOI: 10.1039/C4DT00438H**

**Description**

**Technical Field**

The present invention relates to a dental composition, more particularly to a dental hydraulic composition including hydraulic cement and a non-aqueous liquid.

**Background Art**

Damage to tooth enamel begins due to acids generated through the decomposition of sugar components by bacteria living in the oral cavity to thus cause tooth decay, which is referred to as dental caries. When dental caries are confined to the enamel, little pain is experienced, but when the caries progress to the dentin or pulp, severe pain may result. In this case, root-canal treatment for eliminating the pulp in the root canal has to be performed. When root-canal treatment is performed, it is necessary to fill the empty space in the root canal to suppress the occurrence of secondary infection, Gutta-percha and a sealer being the most widely used materials for filling the empty space in the root canal.

Gutta-percha, which is a very safe material for use in living bodies, has been used for a long time, but is a solid material and thus has to be loaded using heat and pressure, making it difficult to completely fill the convoluted root canal therewith. Hence, various ointment-type sealers have also been used to offset the disadvantages of Gutta-percha. Examples of sealers used to date include resin, zinc oxide, calcium hydroxide, silicone and the like, but have defects of low biosafety or poor adhesion to a tooth structure or Gutta-percha.

Mineral trioxide aggregate (MTA) was first introduced in 1992 by Mahmoud Torabinejad, a professor at the University of Roma Linda in the USA, and has come to be widely used as a dental composition because it is composed of tooth-like components. MTA is composed of Portland cement and bismuth oxide, which is a radiopaque agent. Portland cement comprises dicalcium silicate, tricalcium silicate, tricalcium aluminate, and tricalcium aluminoferrite, and has the property of curing upon contact with water. Calcium silicate is formed into calcium silicate hydrate gel upon contact with water, and furthermore, calcium hydroxide may be formed as a byproduct thereof, thus exhibiting a high pH. Due to the high pH thereof, antibacterial effects are manifested in the oral cavity.

MTA is advantageous because of antibacterial effects and tooth-like components, as described above. Upon application thereof to the tooth, however, the powder, which is used by being mixed with water, begins to react at the time of mixing with water, and thus has the disadvantages of a short working time of less than 5 min and a long curing time of about 4 hr. Furthermore, MTA is problematic in that it appears grayish due to the ferrite component and thus the tooth may be colored, and also in that there is a need to use an additional carrier when applied to a narrow root canal.

KR 2015 0144028 discloses a dental clinic filler derived from a Portland cement clinker.

KR 2010 0037979 discloses a premixed cement paste for use in dental applications, which comprises calcium silicate compounds and an anhydrous liquid carrier.

Therefore, it is necessary to develop a dental hydraulic composition that is easy to use and has a long working time, a short curing time, and improved biocompatibility.

**Disclosure**

**Technical Problem**

Accordingly, the present invention is intended to provide a dental composition.

In addition, the present invention is intended to provide a dental hydraulic composition, which is easy to use and is improved from the aspects of aesthetic appearance, curability and biocompatibility.

**Technical Solution**

An aspect of the present invention provides a dental composition, comprising:

cement; and
a non-aqueous liquid,
in which the cement includes:
a first domain including alite;
a second domain including belite; and
a matrix located between one or more selected from the group consisting of the first domain and the second domain
wherein the matrix includes silicon (Si) -atom-doped tricalcium aluminate ($3CaO \cdot Al_2O_3$).

Also, in the silicon-atom-doped tricalcium aluminate a portion of aluminum atoms of tricalcium aluminate ($3CaO\cdot Al_2O_3$) is substituted with a silicon atom.

Also, the silicon (Si)-doped tricalcium aluminate ($3CaO\cdot Al_2O_3$) is doped with silicon (Si) in an amount of 0.5 to 15 wt%.

Also, the dental composition may further comprise at least one of a radiopaque agent, a calcium phosphate compound, and a curing modifier.

Also, the dental composition may include 100 parts by weight of the cement; 10 to 100 parts by weight of the non-aqueous liquid; and at least one of 20 to 200 parts by weight of the radiopaque agent, 1 to 30 parts by weight of the calcium phosphate compound and 0.1 to 20 parts by weight of the curing modifier.

Also, the cement may be configured such that a weight ratio (D:M) of a weight sum (D, D1 + D2) of the first domain (D1) and the second domain (D2) to a weight of the matrix (M) is 99:1 to 70:30.

Also, the cement may be a hydraulic material prepared by reacting a mixture comprising calcium oxide, silicon dioxide, and aluminum oxide through heat treatment.

Also, the non-aqueous liquid may include at least one selected from among ethanol, propanol, vegetable fat and oil, animal fat and oil, ethylene glycol, propylene glycol, polyethylene glycol, polypropylene glycol, and glycerin, and preferably includes polypropylene glycol, because polypropylene glycol has high biocompatibility compared to polyethylene glycol. When polypropylene glycol is included as the non-aqueous liquid, another non-aqueous liquid other than polypropylene glycol may be further included.

Also, the calcium phosphate compound may include at least one selected from among calcium phosphate, dicalcium phosphate, tricalcium phosphate, tetracalcium phosphate, hydroxyapatite, apatite, octacalcium phosphate, biphasic calcium phosphate, amorphous calcium phosphate, casein phosphopeptide-amorphous calcium phosphate, and bioactive glass.

Also, the calcium phosphate compound may be bioactive glass.

Also, the bioactive glass may be represented by Chemical Formula 1 below.

[Chemical Formula 1]        $(SiO_2)_x(Na_2O)_y(CaO)_z(P_2O_5)_w$

In Chemical Formula 1, x, y, z, and w are numbers of moles, $30\leq x\leq 70$, $0\leq y\leq 40$, $10\leq z\leq 50$, and $1\leq w\leq 10$.

Also, the radiopaque agent may include at least one selected from among zinc oxide, barium sulfate, zirconium oxide, bismuth oxide, barium oxide, iodoform, tantalum oxide, and calcium tungstate.

Also, the curing modifier may include at least one selected from among calcium sulfate dihydrate, calcium sulfate hemihydrate, calcium chloride, and calcium formate.

Also, the dental composition may further comprise a viscosity modifier.

Also, the amount of the viscosity modifier may be 0.1 to 20 parts by weight.

Also, the viscosity modifier may include at least one selected from among cellulose, a cellulose derivative, xanthan gum, polyvinyl alcohol, polyacrylic acid, and polyvinylpyrrolidone.

Also, the dental composition may be in a paste form.

A dental material may be obtained through hydraulic treatment of the above dental composition.

Disclosed but not claimed is a method of preparing a dental composition, comprising manufacturing cement and preparing a composition including the cement and a non-aqueous liquid.

Here, the cement may include a first domain including alite, a second domain including belite, and a matrix located between one or more selected from the group consisting of the first domain and the second domain and configured to include silicon (Si)-atom-doped tricalcium aluminate ($3CaO\cdot Al_2O_3$).

Also, the composition may further comprise at least one of a radiopaque agent and a calcium-phosphate-based compound.

Also, manufacturing the cement may be performed by reacting a mixture comprising calcium oxide, silicon dioxide, and aluminum oxide through heat treatment.

**Advantageous Effects**

According to the present invention, a dental hydraulic composition is provided.

The dental hydraulic composition is provided in the form of a single ointment-type composition and is thus easy to use and is improved from the aspects of aesthetic appearance, curability and biocompatibility.

**Description of Drawings**

FIG. 1 shows the result of SEM-EDS of cement manufactured in Preparation Example 2 of the present invention;

FIG. 2 shows an image of each kind of cement after firing of the mixture prepared in Preparation Example 2 of the present invention and Comparative Preparation Example 3;

FIG. 3 shows an internal image of each kind of cement depending on the cooling conditions after firing of the mixture prepared in Preparation Example 2 of the present invention and Comparative Preparation Example 4;

FIGS. 4a to 4h show the results of X-ray diffraction (XRD) of cement manufactured in each of Preparation Examples 1 to 4 and Comparative Preparation Examples 1 to 4;

FIG. 5 is a graph showing the results of measurement of pH over time in Test Example 3 regarding the composition of Example 3;

FIG. 6 shows SEM images over time after curing of the composition of Example 3 of the present invention;

FIG. 7 shows the results of XRD of the sample surface 4 weeks after curing of the compositions of Examples 1 and 3 of the present invention;

FIG. 8 is images at low and high magnifications showing the interfaces between the composition and dentin and between the composition and GP, after filling the root canal with the composition of Example 3 of the present invention; and

FIG. 9 is a radiographic image showing the state after filling the root canal with the composition of Example 3 of the present invention.

## Best Mode

Hereinafter, embodiments of the present invention are described in detail with reference to the appended drawings so as to be easily performed by a person having ordinary skill in the art to which the present invention belongs.

However, the following description does not limit the present invention to specific embodiments, and moreover, descriptions of known techniques, even if they are pertinent to the present invention, are considered unnecessary and may be omitted insofar as they would make the characteristics of the invention unclear.

The terms herein are used to explain specific embodiments and are not intended to limit the present invention. Unless otherwise stated, the singular expression includes a plural expression. In this specification, the terms "include" or "have" are used to designate the presence of features, numbers, steps, operations, elements, or combinations thereof described in the specification, and should be understood as not excluding the presence or additional possibility of one or more different features, numbers, steps, operations, elements, or combinations thereof.

According to the present invention, a dental hydraulic composition is described below.

An aspect of the present invention pertains to a dental composition, comprising cement and a non-aqueous liquid, in which the cement includes a first domain including alite, a second domain including belite, and a matrix located between one or more selected from the group consisting of the first domain and the second domain wherein the matrix includes silicon (Si)-atom-doped tricalcium aluminate ($3CaO \cdot Al_2O_3$).

The silicon (Si)-doped tricalcium aluminate ($3CaO \cdot Al_2O_3$) is doped with silicon (Si) in an amount of 0.5 to 15 wt%, preferably 1 to 10 wt%, and more preferably 2 to 5 wt%.

The firing temperature of the cement having the domain and matrix structure falls in the range of 1200°C to 1550°C, preferably 1300°C to 1500°C, and more preferably 1400°C to 1500°C. If the firing temperature thereof is lower than 1200°C, belite is mainly formed, rather than alite, undesirably lowering the strength of the cement. On the other hand, if the firing temperature thereof is higher than 1550°C, alite may be formed, but alite, among the components of the cement, may decompose during firing, undesirably lowering the strength of the cement.

The heating rate required to reach the firing temperature of the cement having the domain and matrix structure is 1 °C/min to 20 °C/min, and preferably 2 °C/min to 10 °C/min. If the heating rate required to reach the firing temperature thereof is less than 1 °C/min, the time becomes excessively prolonged, undesirably lowering productivity. On the other hand, if the heating rate required to reach the firing temperature thereof exceeds 20 °C/min, the reaction time between the mixed materials is not sufficient and some materials are left behind and thus the cement strength is lowered, which is undesirable.

The firing time in the temperature range for forming the cement having the domain and matrix structure falls in the range of 0.5 hr to 24 hr, and preferably 1 hr to 12 hr. If the firing time is less than 0.5 hr, the reaction time between the mixed materials is not sufficient and some materials are left behind and thus the cement strength is lowered, which is undesirable. On the other hand, if the firing time exceeds 24 hr, excessive energy consumption is required, thus negating economic benefits, which is undesirable.

The non-aqueous liquid may include at least one selected from among ethanol, propanol, vegetable fat and oil, animal fat and oil, ethylene glycol, propylene glycol, polyethylene glycol, polypropylene glycol, and glycerin.

The non-aqueous liquid is preferably contained in an amount of 10 to 100 parts by weight based on 100 parts by weight of the cement.

The calcium phosphate may include at least one selected from among calcium phosphate, dicalcium phosphate, tricalcium phosphate, tetracalcium phosphate, hydroxyapatite, apatite, octacalcium phosphate, biphasic calcium phosphate, amorphous calcium phosphate, casein phosphopeptide-amorphous calcium phosphate, and bioactive glass.

The calcium phosphate compound may be bioactive glass.

The bioactive glass may be represented by Chemical Formula 1 below.

[Chemical Formula 1]      $(SiO_2)_x(Na_2O)_y(CaO)_z(P_2O_5)_w$

In Chemical Formula 1, x, y, z, and w are numbers of moles, $30 \leq x \leq 70$, $0 \leq y \leq 40$, $10 \leq z \leq 50$, and $1 \leq w \leq 10$.

Calcium hydroxide, generated as a byproduct during hydraulic treatment of the cement, is dissolved in saliva in the oral cavity to thus increase the pH and exhibit an antibacterial effect. Also, it reacts with calcium phosphate to give hydroxyapatite, which is a component of the teeth.

The calcium phosphate is preferably used in an amount of 1 to 30 parts by weight based on 100 parts by weight of the cement.

Also, the dental composition may include a radiopaque agent in order to confirm the results of treatment.

The radiopaque agent may include at least one selected from among zinc oxide, barium sulfate, zirconium oxide, bismuth oxide, barium oxide, iodoform, tantalum oxide, and calcium tungstate.

The radiopaque agent is preferably used in an amount of 20 to 200 parts by weight based on 100 parts by weight of the cement.

Also, the dental composition may further include a viscosity modifier.

The viscosity modifier may include at least one selected from among cellulose, a cellulose derivative, xanthan gum, polyvinyl alcohol, polyacrylic acid, and polyvinylpyrrolidone.

The viscosity modifier is preferably used in an amount of 0.1 to 20 parts by weight based on 100 parts by weight of the cement.

Also, the dental composition may further include a curing modifier.

The curing modifier may include at least one selected from among calcium sulfate dihydrate, calcium sulfate hemihydrate, calcium chloride, and calcium formate.

The curing modifier is preferably used in an amount of 0.1 to 20 parts by weight based on 100 parts by weight of the cement.

Described but not claimed is a dental material obtained through hydraulic treatment of the dental composition of the present invention.

Further described is a method of preparing a dental composition, comprising manufacturing cement and preparing a composition comprising the cement and a non-aqueous liquid.

Here, the cement may include a first domain including alite, a second domain including belite, and a matrix located between one or more selected from the group consisting of the first domain and the second domain and configured to include silicon (Si)-atom-doped tricalcium aluminate ($3CaO \cdot Al_2O_3$).

Also, the composition may further include at least one of a radiopaque agent and a calcium-phosphate-based compound.

Also, the cement may be manufactured by subjecting a mixture comprising calcium oxide, silicon dioxide, and aluminum oxide to reaction through heat treatment and firing.

**Mode for Invention**

[Examples]

A better understanding of the present invention will be given through the following preferred examples, which are merely set forth to illustrate the present invention but are not to be construed as limiting the scope of the present invention.

Preparation Example 1: Ordinary Portland cement (OPC)

141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide, which had been dried at 105°C for 24 hr or more, were weighed, placed in a V-shaped mixer containing ceramic balls having sizes of 10 mm, 5 mm and 1 mm at a predetermined ratio in the same volume as the material mixture in order to realize uniform mixing of materials having different particle sizes and specific gravities and pulverization thereof to a certain size, and then mixed at 50 rpm for 4 hr.

After mixing, the ceramic balls were removed, and the resulting mixture was formed into a hollow cylinder in order to achieve a totally uniform reaction, placed in a platinum crucible, fired in a firing furnace at 1,500°C for 1 hr 30 min, immediately taken out of the firing furnace, and rapidly cooled to 25°C for 10 min using a cooling fan in an ambient atmosphere.

The rapidly cooled cement was primarily pulverized to a size of 1 mm or less under dry conditions, and 1/5 of the volume of a ceramic bottle was filled with the primarily pulverized cement, and ceramic balls having sizes of 10 mm, 5 mm and 1 mm at a predetermined ratio were placed in 1/5 of the volume of the bottle, followed by pulverization for 24 hr. Here, the ceramic bottle and the ceramic balls were used after drying at 105°C for 24 hr or more. The pulverized materials were sieved to an average particle size of 10 $\mu$m or less, yielding a cement powder.

The phase of the powder thus obtained was analyzed using a D/max 2200V/PC, available from Rigaku, Japan. Here, the analysis conditions were 2theta = 20 - 60°, scan speed = 5°/min, target = CuK$\alpha$1, and acceleration voltage of 40 kV and 20 mA.

Preparation Example 2: Cement including silicon-atom-doped matrix

The cement of Preparation Example 2 was manufactured in the same manner as in Preparation Example 1, with the exception that 137 parts by weight of calcium oxide, 45 parts by weight of silicon dioxide and 18 parts by weight of aluminum oxide were used, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide as in Preparation Example 1.

The phase analysis of the cement of Preparation Example 2 was performed in the same manner as the phase analysis of the cement of Preparation Example 1.

Preparation Example 3: Cement including silicon-atom-doped matrix

The cement of Preparation Example 3 was manufactured in the same manner as in Preparation Example 1, with the exception that 141 parts by weight of calcium oxide, 45 parts by weight of silicon dioxide and 14 parts by weight of aluminum oxide were used, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide as in Preparation Example 1.

The phase analysis of the powder of Preparation Example 3 was performed in the same manner as the phase analysis of the powder of Preparation Example 1.

Preparation Example 4: Cement including silicon-atom-doped matrix, silicon dioxide and aluminum oxide

137 parts by weight of calcium oxide, 45 parts by weight of silicon dioxide, and 14 parts by weight of aluminum oxide, which had been dried at 105°C for 24 hr or more, were weighed, placed in a V-shaped mixer containing ceramic balls having sizes of 10 mm, 5 mm and 1 mm at a predetermined ratio in the same volume of the material mixture in order to realize uniform mixing of materials having different particle sizes and specific gravities and pulverization thereof to a certain size, and then mixed at 50 rpm for 4 hr. After completion of mixing, the ceramic balls were removed, and the resulting mixture was formed into a hollow cylinder in order to achieve a totally uniform reaction, placed in a platinum crucible, fired in a firing furnace at 1,500°C for 1 hr 30 min, immediately taken out of the firing furnace, and rapidly cooled to 25°C for 10 min using a cooling fan in an ambient atmosphere. The rapidly cooled cement was pulverized under the same pulverization conditions as in Preparation Example 1, and the cement powder of Preparation Example 4 was further mixed with 1.3 parts by weight of silicon dioxide and 4.5 parts by weight of aluminum oxide. The mixed powder was analyzed in the same manner as in Example 1.

Comparative Preparation Example 1: Cement including silicon dioxide

The cement of Comparative Preparation Example 1 was manufactured in the same manner as in Preparation Example 1, with the exception that 141 parts by weight of calcium oxide and 50.4 parts by weight of silicon dioxide were used, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide as in Preparation Example 1.

The phase analysis of the cement of Comparative Preparation Example 1 was performed in the same manner as the phase analysis of the cement of Preparation Example 1.

Comparative Preparation Example 2: Cement including silicon dioxide

The cement of Comparative Preparation Example 2 was manufactured in the same manner as in Preparation Example 1, with the exception that 151 parts by weight of calcium oxide and 60 parts by weight of silicon dioxide were used and firing at 1,600°C and then air cooling were performed, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide, firing at 1,500°C and then rapid cooling as in Preparation Example 1.

The phase analysis of the cement of Comparative Preparation Example 2 was performed in the same manner as the phase analysis of the cement of Preparation Example 1.

Comparative Preparation Example 3: Cement including silicon dioxide

The cement of Comparative Preparation Example 3 was manufactured in the same manner as in Preparation Example 1, with the exception that 118.8 parts by weight of calcium oxide, 49 parts by weight of silicon dioxide, 10.75 parts by weight of boehmite (aluminum oxide) and 4.61 parts by weight of ferrite oxide (iron oxide) were used and firing at 1,550°C and then air cooling were performed, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide, firing at 1,500°C and then rapid cooling as in Preparation Example 1.

The phase analysis of the cement of Comparative Preparation Example 3 was performed in the same manner as the phase analysis of the cement of Preparation Example 1.

Comparative Preparation Example 4: Cement

The cement of Comparative Preparation Example 4 was manufactured in the same manner as in Preparation Example 1, with the exception that 137 parts by weight of calcium oxide, 45 parts by weight of silicon dioxide and 18 parts by weight of aluminum oxide were used and air cooling was performed, in lieu of using 141.5 parts by weight of calcium oxide, 51.7 parts by weight of silicon dioxide, 2.1 parts by weight of aluminum oxide, 0.7 parts by weight of iron oxide, and 0.6 parts by weight of magnesium oxide and rapid cooling to 25°C for 10 min using a cooling fan in an ambient atmosphere as in Preparation Example 1.

The phase analysis of the cement of Comparative Preparation Example 4 was performed in the same manner as the phase analysis of the cement of Preparation Example 1.

Table 1 below shows the components and amounts of the cement, the firing temperature and the cooling process in Preparation Examples 1 to 4 and Comparative Preparation Examples 1 to 4.

[Table 1]

| No. | Calcium oxide (parts by weight) | Silicon dioxide (parts by weight) | Aluminum oxide (parts by weight) | Iron oxide (parts by weight) | Magnesium oxide (parts by weight) | Firing temp. (°C) | Cooling process and cooling time |
|---|---|---|---|---|---|---|---|
| Preparation Example 1 | 141.5 | 51.7 | 2.1 | 0.7 | 0.6 | 1,500 | Rapid cooling (10 min) |
| Preparation Example 2 | 137.0 | 45.0 | 18.0 | - | - | 1,500 | Rapid cooling (10 min) |
| Preparation Example 3 | 141.0 | 45.0 | 14.0 | - | - | 1,500 | Rapid cooling (10 min) |
| Preparation Example 4 | 137.0 | 45.0 (+ 1.3)[a] | 14.0 (+ 4.5[b]) | - | - | 1,500 | Rapid cooling (10 min) |
| Comparative Preparation Example 1 | 141.0 | 50.4 | - | - | - | 1,500 | Rapid cooling (10 min) |
| Comparative Preparation Example 2 | 151.0 | 60.0 | - | - | - | 1,600 | Air cooling (60 min) |

(continued)

| No. | Calcium oxide (parts by weight) | Silicon dioxide (parts by weight) | Aluminum oxide (parts by weight) | Iron oxide (parts by weight) | Magnesium oxide (parts by weight) | Firing temp. (°C) | Cooling process and cooling time |
|---|---|---|---|---|---|---|---|
| Comparative Preparation Example 3 | 118.8 | 49.0 | 10.75 | 4.61 | - | 1,550 | Air cooling (60 min) |
| Comparative Preparation Example 4 | 137.0 | 45.0 | 18.0 | - | - | 1,500 | Air cooling (60 min) |
| * [a] and [b] designate parts by weight of materials additionally mixed in the fired cement. | | | | | | | |

Example 1: Preparation of dental hydraulic composition

52 parts by weight of the cement manufactured in Preparation Example 2, 20 parts by weight of polypropylene glycol, 25 parts by weight of zirconium oxide and 3 parts by weight of calcium sulfate dihydrate were mixed at 100 rpm for 4 hr using a mixer and allowed to stand for 30 min in a vacuum (-0.095±0.005 MPa) in order to defoam the hydraulic composition and increase the packing density.
Thereafter, a vessel was filled with the hydraulic composition, thereby yielding a dental hydraulic composition.

Example 2: Preparation of dental hydraulic composition

A dental hydraulic composition was prepared in the same manner as in Example 1, with the exception that the cement manufactured in Preparation Example 3 was used in lieu of the cement manufactured in Preparation Example 2.

Example 3: Preparation of dental hydraulic composition

A dental hydraulic composition was prepared in the same manner as in Example 1, with the exception that 50 parts by weight of the cement manufactured in Preparation Example 2 and 2 parts by weight of bioactive glass $((SiO_2)_9(Na_2O)_5(CaO)_5(P_2O_5)_1)$ were used, in lieu of 52 parts by weight of the cement manufactured in Preparation Example 2.

Example 4: Preparation of dental hydraulic composition

A dental hydraulic composition was prepared in the same manner as in Example 3, with the exception that 60 parts by weight of the cement manufactured in Preparation Example 2 and 10 parts by weight of polypropylene glycol were used, in lieu of 50 parts by weight of the cement manufactured in Preparation Example 2 and 20 parts by weight of polypropylene glycol.

Example 5: Preparation of dental hydraulic composition

A dental hydraulic composition was prepared in the same manner as in Example 1, with the exception that 62 parts by weight of the cement manufactured in Preparation Example 2 and 10 parts by weight of polypropylene glycol were used, in lieu of 52 parts by weight of the cement manufactured in Preparation Example 2 and 20 parts by weight of polypropylene glycol.

Example 6: Preparation of dental hydraulic composition

A dental hydraulic composition was prepared in the same manner as in Example 1, with the exception that 52 parts by weight of the cement manufactured in Preparation Example 4 was used, in lieu of 52 parts by weight of the cement manufactured in Preparation Example 2.

Example 7: Preparation of dental hydraulic composition

A dental hydraulic composition was prepared in the same manner as in Example 4, with the exception that 60 parts by weight of the cement manufactured in Preparation Example 2 and 2 parts by weight of tricalcium phosphate were used, in lieu of 60 parts by weight of the cement manufactured in Preparation Example 2 and 2 parts by weight of bioactive glass $((SiO_2)_9(Na_2O)_5(CaO)_5(P_2O_5)_1)$.

Comparative Example 1: Preparation of dental composition

A dental hydraulic composition was prepared in the same manner as in Example 1, with the exception that the ordinary Portland cement manufactured in Preparation Example 1 was used, in lieu of the cement manufactured in Preparation Example 2.

Comparative Example 2: Preparation of dental composition

A dental hydraulic composition was prepared in the same manner as in Example 1, with the exception that the cement manufactured in Comparative Preparation Example 2 was used, in lieu of the cement manufactured in Preparation Example 2.

Comparative Example 3: Preparation of dental composition

A dental hydraulic composition was prepared in the same manner as in Example 1, with the exception that the cement manufactured in Comparative Preparation Example 3 was used, in lieu of the cement manufactured in Preparation Example 2.

Comparative Example 4: Preparation of dental composition

A dental hydraulic composition was prepared in the same manner as in Example 2, with the exception that 52 parts by weight of the cement manufactured in Comparative Preparation Example 4 was used, in lieu of the cement manufactured in Preparation Example 2.

Comparative Example 5: Preparation of dental composition

A dental hydraulic composition was prepared in the same manner as in Example 1, with the exception that the cement manufactured in Comparative Preparation Example 1 was used, in lieu of the cement manufactured in Preparation Example 2.

Table 2 below shows the components and amounts of the dental hydraulic compositions of Examples 1 to 7 and Comparative Examples 1 to 5.

[Table 2]

| | Preparation Example 1 | Preparation Example 2 | Preparation Example 3 | Preparation Example 4 | Comparative Preparation Example 1 | Comparative Preparation Example 2 | Comparative Preparation Example 3 | Comparative Preparation Example 4 | Tricalcium phosphate | Polypropylene glycol | Zirconium oxide | Calcium sulfate dihydrate | Bioactive glass |
|---|---|---|---|---|---|---|---|---|---|---|---|---|---|
| Example 1 | - | 52 | - | - | - | - | - | - | - | 20 | 25 | 3 | - |
| Example 2 | - | - | 52 | - | - | - | - | - | - | 20 | 25 | 3 | - |
| Example 3 | - | 50 | - | - | - | - | - | - | - | 20 | 25 | 3 | 2 |
| Example 4 | - | 60 | - | - | - | - | - | - | - | 10 | 25 | 3 | 2 |
| Example 5 | - | 62 | - | - | - | - | - | - | - | 10 | 25 | 3 | - |
| Example 6 | - | - | - | 52 | - | - | - | - | - | 20 | 25 | 3 | |
| Example 7 | | 60 | | | | | | - | 2 | 10 | 25 | 3 | |
| Comparative Example 1 | 52 | - | - | - | - | - | - | - | - | 20 | 25 | 3 | |
| Comparative Example 2 | - | - | - | - | - | 52 | - | - | - | 20 | 25 | 3 | |
| Comparative Example 3 | - | - | - | - | - | - | 52 | - | - | 20 | 25 | 3 | |

EP 3 593 785 B1

(continued)

| | Preparation Example 1 | Preparation Example 2 | Preparation Example 3 | Preparation Example 4 | Comparative Preparation Example 1 | Comparative Preparation Example 2 | Comparative Preparation Example 3 | Comparative Preparation Example 4 | Tricalcium phosphate | Polypropylene glycol | Zirconium oxide | Calcium sulfate dihydrate | Bioactive glass |
|---|---|---|---|---|---|---|---|---|---|---|---|---|---|
| Comparative Example 4 | - | - | - | - | - | - | - | 52 | | 20 | 25 | 3 | |
| Comparative Example 5 | - | - | - | - | 52 | - | - | - | - | 20 | 25 | 3 | - |

[Test Examples]

Test Example 1: Evaluation of flowability and compressive strength of dental composition

Table 3 below shows the results of evaluation of flowability and compressive strength of the dental compositions of Examples 1 to 7 and Comparative Examples 1 to 5.

For the evaluation of flowability of the dental composition, two glass plates having a size of 40 mm (width) x 40 mm (length), a thickness of about 5 mm and a weight of about 20 g were prepared. 0.05±0.005 ml of the dental composition was placed on the center of one of the glass plates and covered with the other glass plate, to which a weight of 120±2 g was then applied for 10 min. After 10 min, the spread size of the dental composition was measured and measurement values in which the difference between maximum and minimum values was less than 1 mm were recorded, determined three times and averaged.

The compressive strength of the dental composition was determined in a manner in which a gypsum mold having a cavity with a diameter of 4 mm and a height of 6 mm was stored in a constant-temperature and constant-humidity chamber at a temperature of 37±1°C and a humidity of 95%, the cavity thereof was filled with the sample in the constant-temperature and constant-humidity chamber, and the sample was separated from the mold after 1 day, 7 days, 14 days, and 28 days, followed by testing under a force of 100 N at a speed of 1 mm/min using a UTM (Universal Testing Machine), measurement five times and averaging.

[Table 3]

|  | Flowability (mm) | Compressive strength (MPa) | | | |
|---|---|---|---|---|---|
|  |  | 1 day | 7 days | 14 days | 28 days |
| Example 1 | 23.2 | 11.3±1.3 | 30.6±7.4 | 63.9±8.2 | 70.2+5.3 |
| Example 2 | 21.6 | 10.8±1.0 | 29.9±1.2 | 61.4±5.1 | 65.2±3.9 |
| Example 3 | 22.8 | 12.9±0.8 | 34.4±5.4 | 70.7±3.2 | 69.3±1.6 |
| Example 4 | 7.7 | 42.6±1.2 | 107.3±6.6 | 110.6±8.6 | 115.6±8.6 |
| Example 5 | 8.1 | 38.6±1.2 | 85.3±10.6 | 90.6±8.6 | 96.6±6.6 |
| Example 7 | 8.6 | 36.5±1.8 | 80.6±2.3 | 92.6±3.2 | 98.6±3.2 |
| Comparative Example 1 | 22.1 | 7.2±0.6 | 17.3±1.4 | 35.4±5.4 | 40.8±4.2 |
| Comparative Example 2 | 23.3 | 9.2±0.6 | 20.1±1.4 | 50.4±9.4 | 60.8±5.6 |
| Comparative Example 3 | 25.7 | 6.2±0.5 | 10.5±1.5 | 25.9±6.5 | 32.6±6.8 |
| Comparative Example 4 | 21.1 | 9.6±2.8 | 22.9±5.9 | 50.6+1.5 | 53.5±2.2 |
| Comparative Example 5 | 22.2 | 10.2±0.6 | 27.3±1.4 | 62.9±5.4 | 68.8±4.2 |

As is apparent from the results of evaluation of flowability, the flowability varied depending on the amount of cement in the dental composition, and in Examples 1, 2 and 3, the amount of cement in the dental composition was smaller than that in Examples 4, 5 and 7, and thus high flowability was exhibited. As such, the dental composition having high flowability may be used as a sealer, and the dental composition having low flowability may be used for repair.

Based on the results of measurement of compressive strength, the dental composition of Example 4 exhibited the highest compressive strength, and the dental compositions of Examples 5 and 7 showed high compressive strength compared to the examples prepared under other conditions.

Test Example 2: Confirmation of components of cement

Test Example 2-1: Cement including silicon-doped matrix according to the present invention

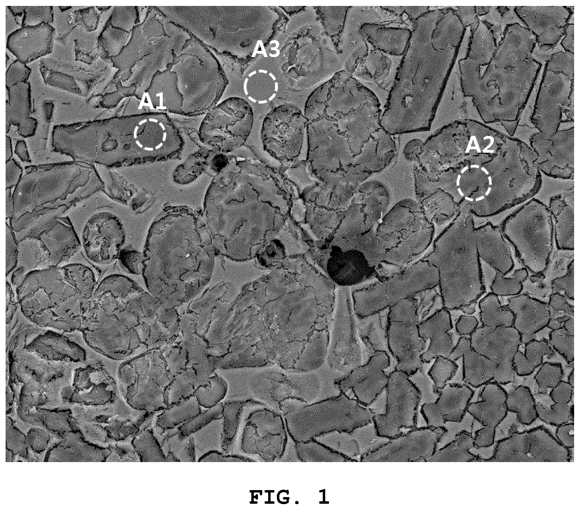

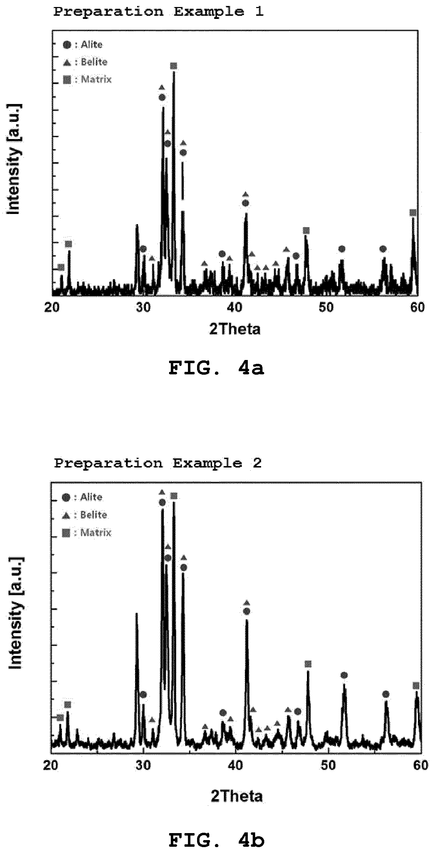

FIG. 1 shows the result of SEM-EDS of the cross-section of the cement manufactured in Preparation Example 2. In FIG. 1, individual examples of portions corresponding to a first domain, a second domain, and a matrix were represented as A1, A2, and A3, respectively. FIGS. 4a to 4h show the results of X-ray diffraction (XRD) of the cement of each of Preparation Examples 1 to 4 and Comparative Preparation Examples 1 to 4. Also, Table 4 below shows the weights of elements of the compositions of the first domain A1, the second domain A2, and the matrix A3 in the cement of Preparation Example 2, as represented in FIG. 1.

[Table 4]

| Classification | FIG. 1 | Ca (wt%) | Si (wt%) | A1 (wt %) | O (wt%) | Total (wt%) |
|---|---|---|---|---|---|---|
| First domain | A1 | 51.23 | 12.05 | 1.77 | 34.95 | 100 |
| Second domain | A2 | 40.62 | 14.51 | 1.84 | 43.03 | 100 |
| Matrix | A3 | 38.64 | 3.48 | 18.6 | 39.28 | 100 |

With reference to FIG. 1 and Table 4, it can be seen that the rectangular alite domain, the circular belite domain, and the silicon-doped tricalcium aluminate matrix were distributed. Based on the results of EDS, the domains and the matrix were confirmed to be composed of calcium, silicon, aluminum and oxygen atoms.

With reference to FIG. 4b, the cement manufactured in Preparation Example 2 was confirmed to include the alite domain, the belite domain and the matrix. As described above, based on the results of EDS, Si and Al were observed to be distributed in the matrix, and the matrix was observed to be a silicon-doped tricalcium aluminate matrix configured such that aluminum of tricalcium aluminate was substituted with silicon.

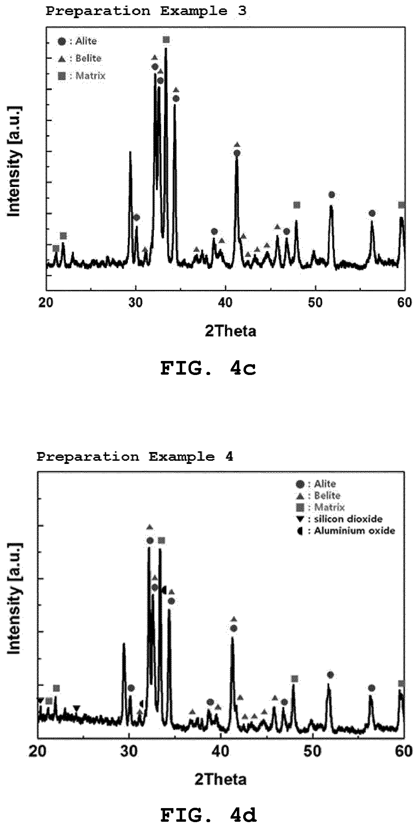

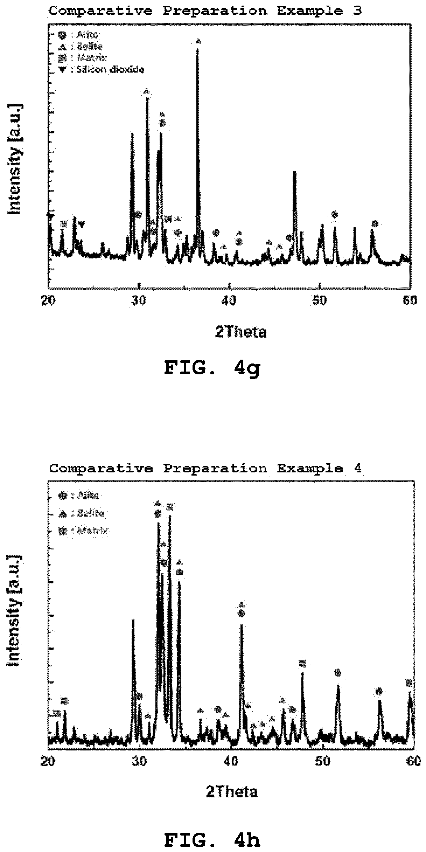

In contrast, with reference to FIGS. 4g and 4d, the cement of each of Comparative Preparation Example 3 and Preparation Example 4 was confirmed to include the alite domain, the belite domain, the matrix and silicon oxide. In the cement manufactured in Comparative Preparation Example 3 and Preparation Example 4, it was confirmed that silicon oxide was formed in a separate phase, without forming the silicon-doped tricalcium aluminate matrix in which aluminum of tricalcium aluminate was substituted with silicon.

Test Example 2-2: Shape of mixture after firing process depending on firing temperature

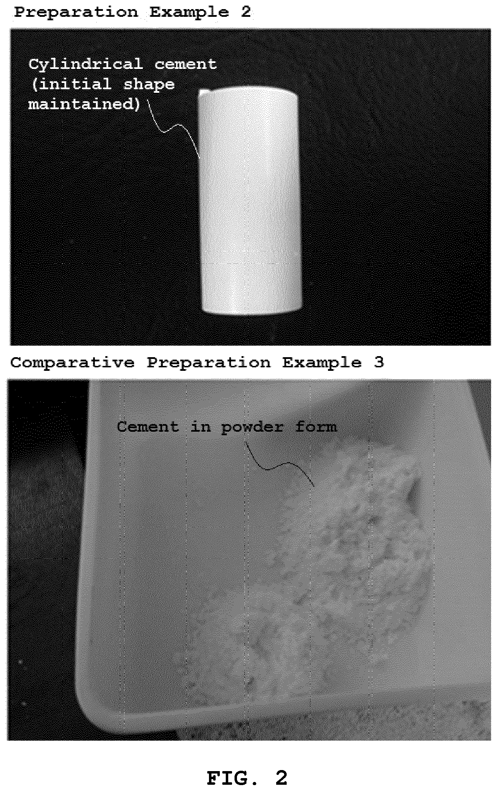

FIG. 2 shows the image of the cement after the process of firing the mixture prepared in each of Preparation Example 2 and Comparative Preparation Example 3.

Even after the mixture of Preparation Example 2 was fired at 1500°C, the initial shape thereof was maintained. However, after firing of the mixture of Comparative Preparation Example 3 at 1550°C, the initial shape thereof disappeared and the decomposed powder shape was observed.

Thus, the shape of the mixture after firing varied depending on the temperature at which the mixture was fired.

Test Example 2-3: Internal image of cement depending on cooling conditions

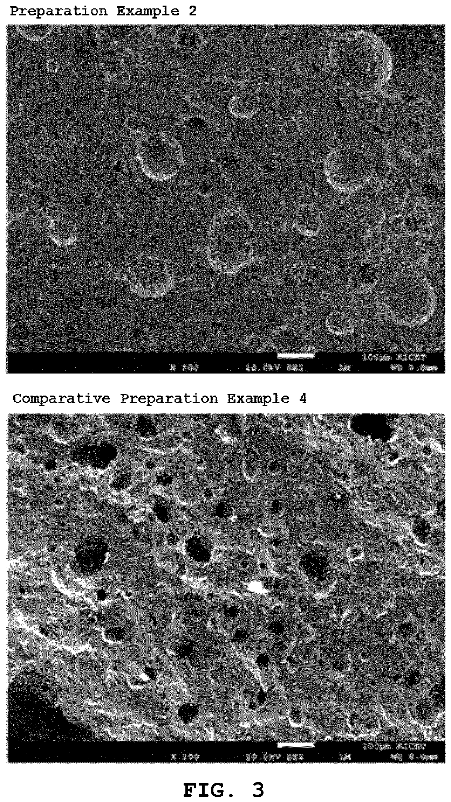

FIG. 3 shows internal images of the cement (rapid cooling) manufactured in Preparation Example 2 of the present invention and of the cement (air cooling) manufactured in Comparative Preparation Example 4.

The internal shape of the cement manufactured in Preparation Example 2 was different from that of the cement manufactured in Comparative Preparation Example 4.

This means that the internal structure varied depending on the cooling conditions even when the same material was used.

Test Example 2-4: X-ray diffraction (XRD) analysis

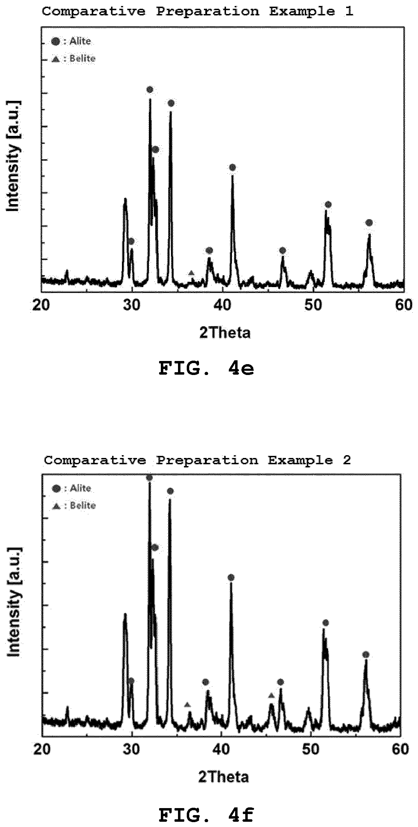

FIGS. 4a to 4d and FIGS. 4e to 4h show the results of X-ray diffraction (XRD) of the cement of each of Preparation Examples 1 to 4 and Comparative Preparation Examples 1 to 4.

With reference to FIG. 4a, the cement manufactured in Preparation Example 1 was confirmed to include the alite domain, the belite domain and the matrix phase.

With reference to FIGS. 4b and 4c, the cement of each of Preparation Examples 2 and 3 was confirmed to include the alite domain, the belite domain and the matrix phase. Specifically, as shown in FIG. 4b, the alite domain, the belite domain, and the matrix were formed using the composition of Preparation Example 2, the proportions of distribution of which were as follows: about 58 vol% of the alite domain, about 18 vol% of the belite domain and about 24 vol% of the matrix. Moreover, the specific proportions thereof were as follows: 84.2 parts by weight of calcium oxide and 30.0 parts by weight of silicon dioxide participated in the formation of the alite domain, 23.0 parts by weight of calcium oxide and 12.4 parts by weight of silicon dioxide participated in the formation of the belite domain, and 29.7 parts by weight of calcium oxide and 18 parts by weight of aluminum oxide participated in the formation of the matrix structure. In addition to the components participating in the structure formation, 2.1 parts by weight of silicon dioxide remained. If silicon dioxide not participating in the structure formation is present alone, the peak for silicon dioxide is observed upon XRD. As shown in the results of FIG. 4b, no peak was observed for silicon dioxide, from which it can be inferred that the silicon atom of remaining silicon dioxide substitutes for aluminum having a similar atom size in the tricalcium aluminate matrix to thus be distributed in the matrix. As for the cement of Preparation Example 2, it was confirmed that the alite domain, the belite domain and the silicon-doped tricalcium aluminate matrix were formed.

With reference to FIG. 4d, the cement manufactured in Preparation Example 4 appeared to have the alite domain and the belite domain, in which some added silicon dioxide and aluminum oxide phases were observed.

With reference to FIG. 4e, the cement manufactured in Comparative Preparation Example 1 appeared to have the alite domain and a small amount of the belite domain.

With reference to FIG. 4f, the cement manufactured in Comparative Preparation Example 2 appeared to have the alite domain and a relatively large amount of the belite domain compared to FIG. 4e.

With reference to FIG. 4g, the cement manufactured in Comparative Preparation Example 3 appeared to have the silicon dioxide phase, in addition to the alite domain, the belite domain and the matrix structure, due to the high firing temperature and the low cooling rate. Furthermore, the belite structure was observed as the main peak, and the amount of the alite domain structure was reduced. Also, based on the results of XRD, the presence of silicon dioxide was deemed to be because silicon resulting from the decomposition of the alite domain or not participating in the structure formation binds to oxygen and is thus provided in the form of stable silicon dioxide, which is observed through XRD. The presence of stable silicon dioxide makes it difficult to dope the matrix structure with silicon, and thus it can be confirmed that the silicon-doped tricalcium aluminate was absent from the matrix of Comparative Preparation Example 3.

With reference to FIG. 4h, although the alite domain, the belite domain and the matrix appeared in the cement manufactured in Comparative Preparation Example 4, the peak of the belite phase was increased in Comparative Preparation Example 4 due to the low cooling rate compared to the XRD data of Preparation Example 2 of FIG. 4b. This is deemed to be because the phase distribution changed depending on the cooling process even under the same firing conditions. Based on the XRD results shown in FIG. 4h, the phase distribution proportions were as follows: about 47 vol% of the alite domain, about 29 vol% of the belite domain and about 24 vol% of the matrix. Moreover, the specific proportions thereof were as follows: 69.6 parts by weight of calcium oxide and 24.8 parts by weight of silicon dioxide participated in the formation of the alite domain, 37.7 parts by weight of calcium oxide and 20.2 parts by weight of silicon dioxide participated in the formation of the belite domain, and 29.7 parts by weight of calcium oxide and 18.0 parts by weight of aluminum oxide participated in the formation of the matrix structure. In Comparative Preparation Example 4, all of the components participated in the formation of the alite domain, the belite domain and the matrix, and thus there were no silicon atoms distributed in the matrix, as in Preparation Example 2, meaning that silicon-doped tricalcium aluminate was absent from the matrix of Comparative Preparation Example 4.

Test Example 3: Evaluation of curing time

In accordance with ISO 6876:2012, evaluation was performed through the following method. Specifically, a gypsum mold having a cavity with a diameter of 10 mm and a height of 1 mm was stored in a constant-temperature and constant-humidity chamber at a temperature of $37\pm1°C$ and a humidity of 95%, and the cavity thereof was filled with the sample in the constant-temperature and constant-humidity chamber. During curing, a Gilmore needle having a weight of $100\pm5$ g and a tip diameter of $2\pm0.1$ mm was placed on the surface of the sample for 15 sec, which was repeated until no mark caused thereby was observed with the naked eye. The time at which no mark was observed was determined to be the curing time, and was measured three times and averaged. The results are shown in Table 5 below.

[Table 5]

| No. | Curing time |
| --- | --- |
| Example 1 | 18 min 30 sec |
| Example 2 | 30 min 15 sec |
| Example 3 | 18 min |
| Example 4 | 15 min 10 sec |
| Example 5 | 17 min |
| Example 7 | 20 min |
| Comparative Example 1 | 150 min |
| Comparative Example 2 | 40 min 50 sec |

(continued)

| No. | Curing time |
|---|---|
| Comparative Example 3 | 170 min |
| Comparative Example 4 | 35 min 20 sec |
| Comparative Example 5 | 23 min 20 sec |

As is apparent from Table 5, the curing time of the dental hydraulic compositions of Examples 1 to 5 and 7 was faster than the curing time of the dental hydraulic compositions of Comparative Examples 1 to 5.

The reason why the curing time of the dental hydraulic composition of Comparative Example 2 was long was that the amount of belite having low curing reactivity was relatively high compared to Comparative Example 5 and thus affected the curing time.

The reason why the curing time of the dental hydraulic composition of Comparative Example 3 was long was that the materials decomposed during the firing process impeded the curing reaction and thus affected the curing time.

The reason why the curing time of the dental hydraulic composition of Comparative Example 4 was long was that the amount of belite that was produced in the cement under air cooling conditions was large compared to the cement manufactured under rapid cooling conditions, and thus the curing reaction rate was decreased compared to the cement composition used in Example 1, thus affecting the curing time.

Test Example 4: Evaluation of pH

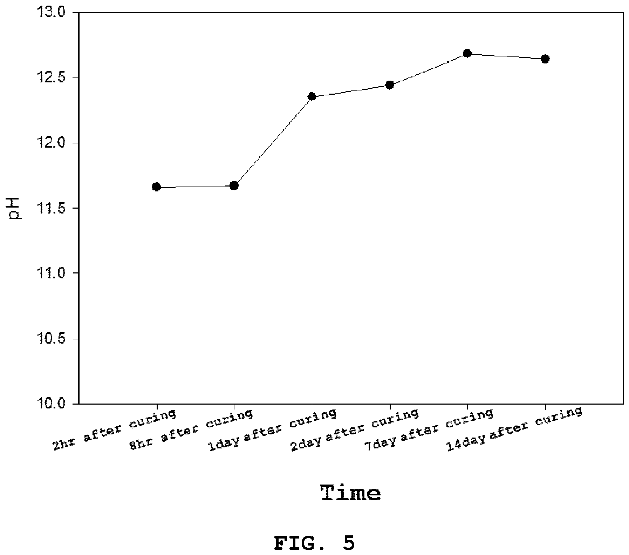

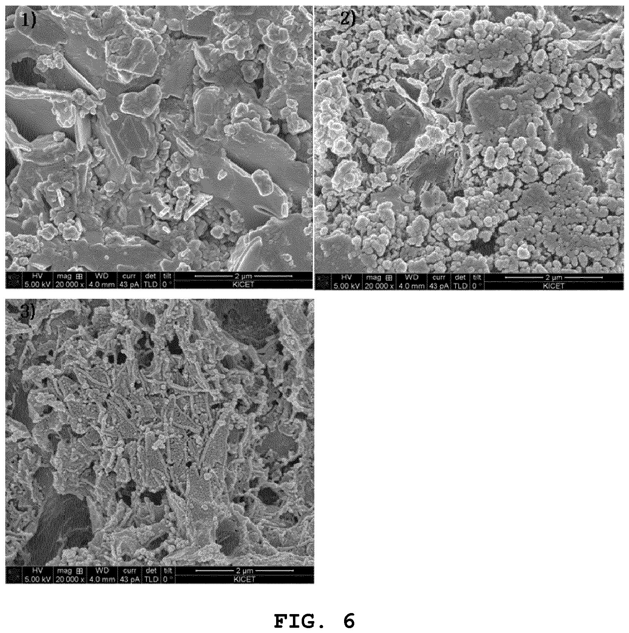

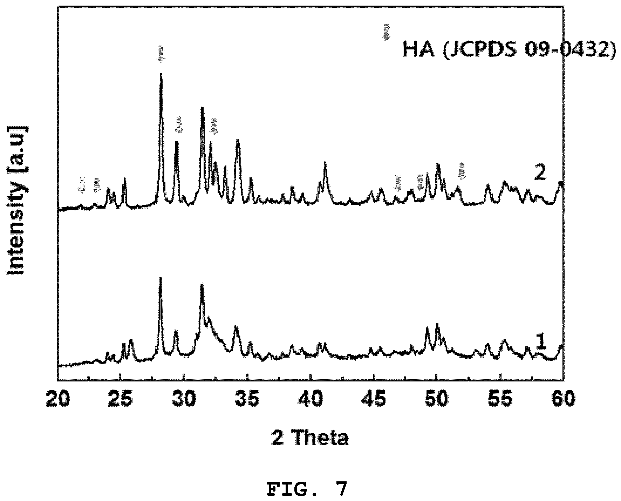

FIG. 5 is a graph showing the results of measurement of pH over time in Test Example 3 regarding the composition of Example 3, FIG. 6 shows SEM images over time after curing of the composition of Example 3 according to the present invention, and FIGS. 7(1) and (2) show the results of XRD of the sample surface 4 weeks after curing of the compositions of Examples 1 and 3 according to the present invention.

With reference to FIG. 5, a mold having a diameter of $10\pm2$ mm and a height of $2\pm1$ mm was filled with the sample of Example 3 to afford a test specimen, which was then placed in 20 ml of distilled water, and the pH thereof was measured over time.

Test Example 5: Evaluation of ability to form hydroxyapatite

In accordance with ISO 23317:2014, evaluation was performed through the following method.

1) Preparation of stimulated body fluid (SBF)

700 ml of distilled water and magnetic bars were placed in a 1 L plastic beaker and the beaker was covered with transparent glass or a wrap. A water bath was placed on a magnetic stirrer and the beaker was placed therein. During stirring, heat was applied until the temperature of the water bath was $36.5\pm1.5°C$. 8.035 g of NaCl, 0.355 g of NaHCO$_3$, 0.225 g of KCl, 0.231 g of K$_2$HPO$_4$3H$_2$O, 0.311 g of MgCl$_2$6H$_2$O, 39 ml of 1 mol/L HCl, 0.292 g of CaCl$_2$, and 0.072 g of Na$_2$SO$_4$ were dissolved with stirring one by one. As such, if the amount of the solution was less than 0.9 L, distilled water was added to make 0.9 L. When the temperature of the solution was measured and thus reached $36.5\pm1.5°C$, tris was added little by little and changes in pH were observed. When the pH of the solution was $7.45\pm0.01$, the addition of tris was stopped and an HCl solution was added. The HCl solution was added until the pH was $7.42\pm0.01$. When the pH was less than $7.42\pm0.01$, the remaining tris was dissolved little by little. The pH was adjusted to the range of 7.42 to 7.45, and tris was completely dissolved and then HCl was added little by little such that the pH of the solution was $7.42\pm0.01$. The prepared solution was placed in a 1 L volumetric flask, distilled water was added to the 1 L marking line of the flask, and the flask was placed in a water bath so that the solution temperature was decreased to less than 20°C. When the temperature became less than 20°C, distilled water was added to the 1 L marking line of the flask.

2) Test method

A mold having a diameter of $10\pm2$ mm and a height of $2\pm1$ mm was filled with the sample of each of Examples 1 and 3 to give a test specimen, the surface area (Sa) of which was then calculated. The volume of SBF (Vs) necessary for the test was calculated using Equation 1 below.

$$[Equation\ 1]$$

$$Vs = 100\ mm \times Sa$$

The calculated volume of SBF was placed in a plastic vessel with a lid. Heat was applied until the temperature of the SBF was 36.5°C, and the test specimen was placed therein. Here, the test specimen has to be completely immersed in SBF. The SBF containing the test specimen therein was stored at 36.5°C, and the test specimen was periodically taken out and washed with water. The washed test specimen was dried in a desiccator at room temperature. The surface of the dried test specimen was observed through SEM and XRD.

With reference to the SEM images of FIG. 6, the formation of small particles on the sample surface immediately after curing, after 2 weeks and after 4 weeks was confirmed. This means that hydroxyapatite was formed. Specifically, with reference to the XRD data (after 4 weeks) of FIG. 7, the ability to form hydroxyapatite was increased when bioactive glass was used.

With reference to FIGS. 5 and 6, the dental cement according to the present invention exhibited significantly increased ability to form hydroxyapatite when containing bioactive glass.

Test Example 6: Clinical test for root-canal filling

A syringe for use in root-canal treatment was filled with the dental composition of Example 3 of the present invention so as to prevent foaming and was equipped with a dispensing tip so that the root canal was filled therewith.

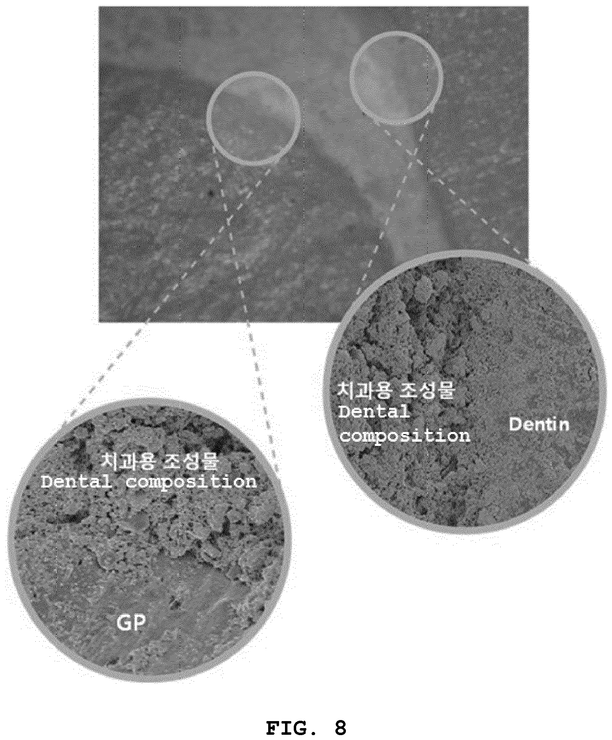

With reference to FIG. 8, as shown in the cross-sectional image of the tooth filled with the composition of Example 3, attachment to the surface of a Gutta-percha point (GP) and to the dentin of the tooth was good.

If the interface between the dentin and the dental composition or between the GP and the dental composition is not sealed with the composition, a microleak may develop and secondary caries attributable to tooth bacteria may occur.

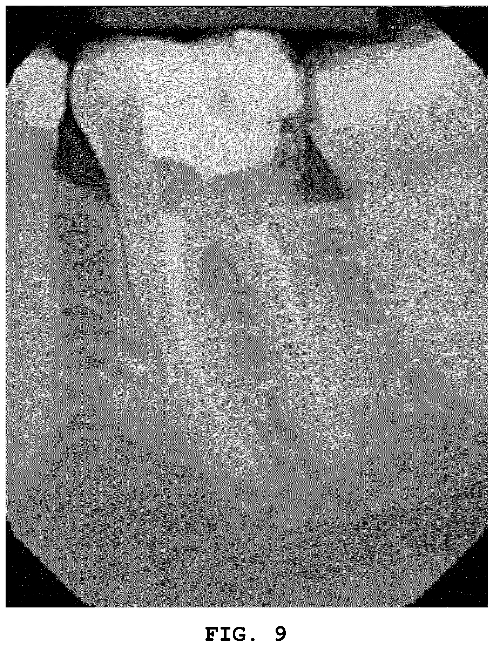

With reference to FIG. 9, the extent to which the root canal was filled with the composition of Example 3 was confirmed through radiometry, indicating that fine portions were efficiently filled. Moreover, good radiopacity of the composition is helpful in obtaining information after treatment.

**Industrial Applicability**

According to the present invention, a dental composition may be a single ointment-type composition comprising cement, a non-aqueous liquid, a radiopaque agent and calcium phosphate, and is thus easy to use and can be improved from the aspects of aesthetic appearance, curability and biocompatibility.

**Claims**

1.  A dental composition, comprising:

> cement; and
> a non-aqueous liquid,
> wherein the cement includes:

>> a first domain including alite;
>> a second domain including belite; and
>> a matrix located between one or more selected from the group consisting of the first domain and the second domain wherein the matrix includes silicon (Si)-atom-doped tricalcium aluminate ($3CaO \cdot Al_2O_3$).

2.  The dental composition of claim 1, wherein in the silicon-atom-doped tricalcium aluminate a portion of aluminum atoms of tricalcium aluminate ($3CaO \cdot Al_2O_3$) is substituted with a silicon atom.

3.  The dental composition of claim 1, wherein the silicon (Si)-doped tricalcium aluminate ($3CaO \cdot Al_2O_3$) is doped with silicon (Si) in an amount of 0.5 to 15 wt%.

4.  The dental composition of claim 1, wherein the dental composition further comprises at least one of a radiopaque agent, a calcium phosphate compound, and a curing modifier, wherein the curing modifier includes at least one selected from among calcium sulfate dihydrate, calcium sulfate hemihydrate, calcium chloride, and calcium formate.

5. The dental composition of claim 4, wherein the dental composition comprises:

> 100 parts by weight of the cement;
> 10 to 100 parts by weight of the non-aqueous liquid; and
> at least one of 20 to 200 parts by weight of the radiopaque agent, 1 to 30 parts by weight of the calcium phosphate compound, and 0.1 to 20 parts by weight of the curing modifier.

6. The dental composition of claim 1, wherein the non-aqueous liquid includes at least one selected from among ethanol, propanol, vegetable fat and oil, animal fat and oil, ethylene glycol, propylene glycol, polyethylene glycol, polypropylene glycol, and glycerin.

7. The dental composition of claim 6, wherein the non-aqueous liquid includes polypropylene glycol.

8. The dental composition of claim 1, wherein the non-aqueous liquid includes polypropylene glycol, and further includes at least one selected from among ethanol, propanol, vegetable fat and oil, animal fat and oil, ethylene glycol, propylene glycol, polyethylene glycol, and glycerin.

9. The dental composition of claim 4, wherein the calcium phosphate compound includes at least one selected from among calcium phosphate, dicalcium phosphate, tricalcium phosphate, tetracalcium phosphate, hydroxyapatite, apatite, octacalcium phosphate, biphasic calcium phosphate, amorphous calcium phosphate, casein phosphopeptide-amorphous calcium phosphate, and bioactive glass.

10. The dental composition of claim 9, wherein the calcium phosphate compound is bioactive glass.

11. The dental composition of claim 10, wherein the bioactive glass is represented by Chemical Formula 1 below:

$$[\text{Chemical Formula 1}] \qquad (SiO_2)_x(Na_2O)_y(CaO)_z(P_2O_5)_w$$

in Chemical Formula 1, x, y, z, and w are numbers of moles, $30 \leq x \leq 70$, $0 \leq y \leq 40$, $10 \leq z \leq 50$, and $1 \leq w \leq 10$.

12. The dental composition of claim 4, wherein the radiopaque agent includes at least one selected from among zinc oxide, barium sulfate, zirconium oxide, bismuth oxide, barium oxide, iodoform, tantalum oxide, and calcium tungstate.

13. The dental composition of claim 5, wherein the dental composition further comprises 0.1 to 20 parts by weight of a viscosity modifier, wherein the viscosity modifier includes at least one selected from among cellulose, a cellulose derivative, xanthan gum, polyvinyl alcohol, polyacrylic acid, and polyvinylpyrrolidone.

**Patentansprüche**

1. Eine Dentalzusammensetzung, umfassend:

> Zement; und
> eine nicht-wässrige Flüssigkeit,
> wobei der Zement enthält:
>
> > eine erste Domäne, die Alit enthält;
> > eine zweite Domäne, die Belit enthält; und
> > eine Matrix, die angeordnet ist zwischen einer oder mehreren, ausgewählt aus der Gruppe bestehend aus der ersten Domäne und der zweiten Domäne, wobei die Matrix mit Silizium (Si)-Atomen dotiertes Tricalciumaluminat ($3CaO \cdot Al_2O_3$) enthält.

2. Dentalzusammensetzung nach Anspruch 1, wobei in dem mit Siliziumatomen dotierten Tricalciumaluminat ein Teil der Aluminiumatome des Tricalciumaluminats ($3CaO \cdot Al_2O_3$) durch ein Siliziumatom ersetzt ist.

3. Dentalzusammensetzung nach Anspruch 1, wobei das Silizium (Si)-dotierte Tricalciumaluminat ($3CaO \cdot Al_2O_3$) mit

Silizium (Si) in einer Menge von 0,5 bis 15 Gew.-% dotiert ist.

4. Dentalzusammensetzung nach Anspruch 1, wobei die Dentalzusammensetzung des Weiteren mindestens eines von einem röntgenopaken Mittel, einer Calciumphosphatverbindung und einem Aushärtungsmodifizierungsmittel umfasst,
wobei das Aushärtungsmodifizierungsmittel mindestens eines, ausgewählt aus Calciumsulfat-Dihydrat, Calciumsulfat-Halbhydrat, Calciumchlorid und Calciumformiat, enthält.

5. Dentalzusammensetzung nach Anspruch 4, wobei die Dentalzusammensetzung umfasst:

100 Gew.-Teile des Zements;
10 bis 100 Gewichtsteile der nicht-wässrigen Flüssigkeit; und
mindestens eines aus 20 bis 200 Gewichtsteile des röntgenopaken Mittels, 1 bis 30 Gewichtsteile der Calciumphosphatverbindung und 0,1 bis 20 Gewichtsteile des Aushärtungsmodifizierungsmittels.

6. Dentalzusammensetzung nach Anspruch 1, wobei die nicht-wässrige Flüssigkeit mindestens eines, ausgewählt aus Ethanol, Propanol, pflanzliches Fett und Öl, tierisches Fett und Öl, Ethylenglykol, Propylenglykol, Polyethylenglykol, Polypropylenglykol und Glycerin, enthält.

7. Dentalzusammensetzung nach Anspruch 6, wobei die nicht-wässrige Flüssigkeit Polypropylenglykol enthält.

8. Dentalzusammensetzung nach Anspruch 1, wobei die nichtwässrige Flüssigkeit Polypropylenglykol enthält und außerdem mindestens eines, ausgewählt aus Ethanol, Propanol, pflanzliches Fett und Öl, tierisches Fett und Öl, Ethylenglykol, Propylenglykol, Polyethylenglykol und Glycerin, enthält.

9. Dentalzusammensetzung nach Anspruch 4, wobei die Calciumphosphatverbindung mindestens eines, ausgewählt aus Calciumphosphat, Dicalciumphosphat, Tricalciumphosphat, Tetracalciumphosphat, Hydroxyapatit, Apatit, Octacalciumphosphat, biphasischem Calciumphosphat, amorphem Calciumphosphat, Caseinphosphopeptidamorphem Calciumphosphat und bioaktivem Glas, enthält.

10. Dentalzusammensetzung nach Anspruch 9, wobei die Calciumphosphatverbindung bioaktives Glas ist.

11. Dentalzusammensetzung nach Anspruch 10, wobei das bioaktive Glas durch die nachstehende chemische Formel 1 dargestellt wird:

[Chemische Formel 1] $(SiO_2)_x(Na_2O)_y(CaO)_z(P_2O_5)_w$

wobei in der chemischen Formel 1 x, y, z und w Molzahlen sind, und $30 \leq x \leq 70$, $0 \leq y \leq 40$, $10 \leq z \leq 50$ und $1 \leq w \leq 10$ gilt.

12. Dentalzusammensetzung nach Anspruch 4, wobei das röntgenopake Mittel mindestens eines, ausgewählt aus Zinkoxid, Bariumsulfat, Zirkoniumoxid, Wismutoxid, Bariumoxid, Jodoform, Tantaloxid und Calciumwolframat, enthält.

13. Dentalzusammensetzung nach Anspruch 5, wobei die Dentalzusammensetzung des Weiteren 0,1 bis 20 Gewichtsteile eines Viskositätsmodifizierungsmittels umfasst, wobei das Viskositätsmodifizierungsmittel mindestens eines, ausgewählt aus Cellulose, einem Cellulosederivat, Xanthangummi, Polyvinylalkohol, Polyacrylsäure und Polyvinylpyrrolidon, enthält.

## Revendications

1. Composition dentaire, comprenant :

du ciment ; et
un liquide non aqueux,
dans laquelle le ciment inclut :

un premier domaine incluant de l'alite ;

un deuxième domaine incluant de la bélite ; et

une matrice située entre un ou plusieurs éléments choisis parmi le groupe constitué du premier domaine et du second domaine, dans laquelle la matrice inclut de l'aluminate tricalcique dopé aux atomes de silicium (Si) ($3CaO \cdot Al_2O_3$).

2. Composition dentaire de la revendication 1, dans laquelle dans l'aluminate tricalcique dopé aux atomes de silicium, une portion des atomes d'aluminium de l'aluminate tricalcique ($3CaO \cdot Al_2O_3$) est substituée par un atome de silicium.

3. Composition dentaire de la revendication 1, dans laquelle l'aluminate tricalcique dopé au silicium (Si) ($3CaO \cdot Al_2O_3$) est dopé avec du silicium (Si) dans une proportion de 0,5 à 15 % en poids.

4. Composition dentaire de la revendication 1, dans laquelle la composition dentaire comprend en outre au moins un agent radio-opaque, un composé de phosphate de calcium et un modificateur de durcissement,

dans laquelle le modificateur de durcissement inclut au moins un élément choisi parmi le dihydrate de sulfate de calcium, le hémihydrate de sulfate de calcium, le chlorure de calcium et le formiate de calcium.

5. Composition dentaire de la revendication 4, dans laquelle la composition dentaire comprend :

100 parties en poids du ciment ;

10 à 100 parties en poids du liquide non aqueux ; et

au moins un élément parmi 20 à 200 parties en poids de l'agent radio-opaque, 1 à 30 parties en poids du composé de phosphate de calcium, et 0,1 à 20 parties en poids du modificateur de durcissement.

6. Composition dentaire de la revendication 1, dans laquelle le liquide non aqueux inclut au moins un élément choisi parmi l'éthanol, le propanol, les graisses et huiles végétales, les graisses et huiles animales, l'éthylène glycol, le propylène glycol, le polyéthylène glycol, le polypropylène glycol et la glycérine.

7. Composition dentaire de la revendication 6, dans laquelle le liquide non aqueux inclut du polypropylène glycol.

8. Composition dentaire de la revendication 1, dans laquelle le liquide non aqueux inclut du polypropylène glycol, et inclut en outre au moins un élément choisi parmi l'éthanol, le propanol, les graisse et les huiles végétales, les graisses et les huiles animales, l'éthylène glycol, le propylène glycol, le polyéthylène glycol et la glycérine.

9. Composition dentaire de la revendication 4, dans laquelle le composé de phosphate de calcium inclut au moins un élément choisi parmi le phosphate de calcium, le phosphate dicalcique, le phosphate tricalcique, le phosphate tétracalcique, l'hydroxyapatite, l'apatite, le phosphate octacalcique, le phosphate de calcium biphasique, le phosphate de calcium amorphe, le phosphopeptide de caséine-phosphate de calcium amorphe, et le verre bioactif.

10. Composition dentaire de la revendication 9, dans laquelle le composé de phosphate de calcium est du verre bioactif.

11. Composition dentaire de la revendication 10, dans laquelle le verre bioactif est représenté par la formule chimique 1 ci-dessous :

[Formule chimique 1] $(SiO_2)_x(Na_2O)_y(CaO)_z(P_2O_5)_w$

dans la formule chimique 1, x, y, z et w sont des nombres de moles, $30 \leq x \leq 70$, $0 \leq y \leq 40$, $10 \leq z \leq 50$, et $1 \leq w \leq 10$.

12. Composition dentaire de la revendication 4, dans laquelle l'agent radio-opaque inclut au moins un élément choisi parmi l'oxyde de zinc, le sulfate de baryum, l'oxyde de zirconium, l'oxyde de bismuth, l'oxyde de baryum, l'iodoforme, l'oxyde de tantale et le tungstate de calcium.

13. Composition dentaire de la revendication 5, dans laquelle la composition dentaire comprend en outre 0,1 à 20 parties en poids d'un modificateur de viscosité, dans laquelle le modificateur de viscosité inclut au moins un élément choisi parmi la cellulose, un dérivé de la cellulose, la gomme xanthane, l'alcool polyvinylique, l'acide polyacrylique et la polyvinylpyrrolidone.

FIG. 1

Preparation Example 2

Cylindrical cement
(initial shape
maintained)

Comparative Preparation Example 3

Cement in powder form

# FIG. 2

Preparation Example 2

Comparative Preparation Example 4

FIG. 3

Preparation Example 1

FIG. 4a

Preparation Example 2

FIG. 4b

Preparation Example 3

FIG. 4c

Preparation Example 4

FIG. 4d

Comparative Preparation Example 1

FIG. 4e

Comparative Preparation Example 2

FIG. 4f

25

FIG. 4g

FIG. 4h

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20150144028
- KR 20100037979